# EUROPEAN PATENT SPECIFICATION

(11) **EP 2 182 073 B1**
(45) Date of publication and mention of the grant of the patent: **16.04.2014**
(21) Application number: 08291017.5
(22) Date of filing: 30.10.2008
(51) Int. Cl.: C12Q 1/37, G01N 33/50, A61P 35/00

(54) **Ubiquitin specific proteases responsible for MCL-1 stability and uses thereof**
Ubiquitinspezifische Proteasen für die MCL-1-Stabilität und Verwendungen davon
Protéases spécifiques de l'ubiquitine responsables de la stabilité MCL-1 et leurs utilisations

(43) Date of publication of application: 05.05.2010
(73) Proprietor: Les Laboratoires Servier, 92284 Suresnes Cedex (FR); Hybrigenics, 75014 Paris (FR)
(72) Inventor: Geneste, Olivier, 92500 Rueil Malmaison (FR); Hickman, John, 75017 Paris (FR); Colland, Frédéric, 95380 Puiseux-en-France (FR); Daviet, Laurent, 74100 Vétraz-Monthoux (FR)
(74) Representative: Bestel, Delphine

(56) References cited:
- WO-A-03/100000
- WO-A-2008/011601
- US-A1- 2006 040 335
- DAVIET ET AL: "Targeting ubiquitin specific proteases for drug discovery" BIOCHIMIE, MASSON, PARIS, FR, vol. 90, no. 2, 29 January 2008 (2008-01-29), pages 270-283, XP022438301 ISSN: 0300-9084
- WARR MATTHEW R ET AL: "Unique biology of Mcl-1: therapeutic opportunities in cancer" CURRENT MOLECULAR MEDICINE (HILVERSUM), vol. 8, no. 2, March 2008 (2008-03), pages 138-147, XP002522774 ISSN: 1566-5240
- PAULSSON K ET AL: "A novel and cytogenetically cryptic t(7;21)(p22;q22) in acute myeloid leukemia results in fusion of RUNX1 with the ubiquitin-specific protease gene USP42" LEUKEMIA (BASINGSTOKE), vol. 20, no. 2, February 2006 (2006-02), pages 224-229, XP002522775 ISSN: 0887-6924
- HOLTKAMP NIKOLA ET AL: "Characterization of the amplicon on chromosomal segment 4q12 in glioblastoma multiforme." NEURO-ONCOLOGY JUL 2007, vol. 9, no. 3, July 2007 (2007-07), pages 291-297, XP002522776 ISSN: 1522-8517
- WONG Y F ET AL: "Gene expression pattern associated with radiotherapy sensitivity in cervical cancer." CANCER JOURNAL (SUDBURY, MASS.) 2006 MAY-JUN, vol. 12, no. 3, May 2006 (2006-05), pages 189-193, XP008104797 ISSN: 1528-9117

## Description

The present invention relates to a use of a USP polypeptide specific to Mcl-1 selected from the group consisting of USP26, USP38, USP42 or USP46 as a screening tool for an agent for treating cancer.

Myeloid cell leukemia-1 (Mcl-1), originally identified in differentiating myeloid cells, belongs to the anti-apoptotic Bcl-2 family members (Bcl-2, Bd-XL, Bcl-W, A1). Mcl-1 acts at an early step in apoptosis induced by various death stimuli, particularly DNA damages, and serves as a major survival factor. More precisely, Mcl-1, which promotes cell survival by interfering in a cascade of events leading to release of cytochrome c from mitochondria, is a short half-life protein. Processes that regulate Mcl-1 are thus critically important in pathological situations such as cancer.

The degradation of Mcl-1 can be blocked by proteasome inhibitors suggesting a role for the Ubiquitin-Proteasome pathway in apoptosis.

The ubiquitin-mediated degradation is a tightly regulated process where proteins are tagged with ubiquitin moeties through a series of enzymatic reactions involving an E1-activating enzyme, an E2-conjugating enzyme, and an E3 ubiquitin ligase that determines substrate specificity. Proteins are then degraded by the proteasome. The reverse process also occurs and leads to the removal of the ubiquitin chain, i.e. deubiquitination. This latter reaction is performed, in part, by enzymes termed ubiquitin specific proteases (USPs) and results in restoration of substrate to its normal state.

The ubiquitin specific proteases (USPs) belong to the broad family of deubiquitinating enzymes (DUBs). The human genome encodes approximately 95 putative deubiquitinating enzymes (DUBs) which belong to the super-family of proteases. These enzymes are divided into 5 sub-families, among which the most-known sub-classes are USP (Ubiquitin Specific Proteases) and UCH (Ubiquitin C-terminal hydrolases). The 60 human USP proteins are cysteine proteases and their catalytic domain contain a 'Cysteine box' and an 'Histidine Box'. The main function of USP is to remove ubiquitin from specific protein substrates.

Clinical success with proteasome inhibitors strengthens the notion that modulating other steps in Ubiquitin-Proteasome pathways, such as E3s or USP, might be therapeutically successful with more specificity and efficacy.

It has been also reported in a publication the identification of an E3 ligase named Mule/ARF-BP1/UreB1 that is required and sufficient for the poly-ubiquitination of Mcl-1. Interestingly, down-regulation of Mule expression resulted in stabilization and accumulation of Mcl-1, leading to cells becoming more resistant to killing by genotoxic agents.

The use of USP13 as a screening tool for an agent for treating cancer as well as the use of cells transformed with USP13 expression constructs for the same purpose has been described in patent specification WO03/100000.

The results disclosed in the present invention indicate that USPs specific to Mcl-1 exist and would therefore act on the stabilization of cellular Mcl-1 to control apoptosis. Inhibition of these USPs would lead to an increase of Mcl-1 degradation by the proteasome promoting the onset of apoptosis. This observation offers a new way to target Mcl-1 by inhibiting the Mcl-1-regulating USP protein with small molecule compounds.

A first object of the invention is a use of a USP polypeptide specific to Mcl-1 selected from the group consisting of
a) a polypeptide consisting of USP26, USP38, USP42 or USP46;
b) a functional fragment of a polypeptide USP26, USP38, USP42 or USP46 and exhibiting an activity of deubiquitination;
c) a polypeptide comprising an amino acid sequence having a 85% or more homology with a polypeptide consisting of USP26, USP38, USP42 or USP46 and exhibiting an activity of deubiquitination;
as a screening tool for an agent for treating cancer.

The Mcl-1-regulating USPs disclosed in the invention are USP26 (GI:13994267), USP38 (GI: 31559774), USP42 (GI: 79750943), USP46 (GI: 31377708) and can cleave either peptide bonds linking ubiquitin as part of a precursor fusion protein, releasing free ubiquitin moieties or cleave bonds conjugating ubiquitin (post-translationally) to proteins.

In a particular embodiment, the invention provides the use of USP46, a functional fragment or a substantially homologous sequence thereof as a screening tool for an agent for treating cancer.

USP46 is a small 366 AA-long protein of unknown function which contains the UCH domain, a catalytic domain involved in deubiquitinating functions. USP46 was found to be widely distributed in human tissues and to be active in vitro. Recently, an amplicon on chromosome 4q12, containing USP46 as flanking gene, has been described in glioblastomas. USP46 gene amplification was detected in several glioblastoma and glioblastoma cell cultures. USP46 sequence analysis identified a human paralog, USP12=UBH1 (NP_872294), which exhibits 88% identity in protein sequence and 76% identity in nucleotide sequence. Furthermore, the catalytic domain of USP46 and USP12 exhibit 100% identity in protein sequence. Biochemical assay revealed that the mouse UBH1, with 98.3% amino acid identity with human UBH1, exhibits deubiquitinating enzyme activity.

A "functional fragment" retains one or more of the biological activities of the protein such as the ability to hydrolyze peptide bonds with ubiquitin or comprises a domain or motif e.g. catalytic site, USP signature, domain including the cysteine or histidine ubiquitin recognition sites, ubiquitin binding sites, or sites important for carrying out the other functions of the protease.

The terms "homology" and "substantially homologous" when used herein with respect to a sequence mean that a sequence when compared to its corresponding reference sequence, has substantially the same structure and function. When a position in the reference sequence is occupied by the same amino acid the molecules are homologous at that position (i.e. there is identity at that position). The percentage of homology between the substantially homologous sequence and reference sequence desirably is at least 85%, more desirably at least 88%, preferably at least 95%, still more preferably at least 99%.

In a second aspect of the invention, a use of a cell which is transformed with an expression vector comprising a polynucleotide encoding a polypeptide consisting of USP26, USP38, USP42 or USP46, a functional fragment or a substantially homologous sequence thereof exhibiting an activity of deubiquitination is provided. The cell is expressing the polypeptide as a screening tool for an agent for treating cancer.

The expression "screening tool" as used herein means a tool used for screening more particularly a polypeptide or a cell expressing a polypeptide used for screening.

As used herein, the expression "agent for treating cancer" includes not only a drug for curing a patient suffering from cancer, but also a drug for preventing a progress of cancer. "Treating cancer" refers to the prophylactic or preferably therapeutic (including but not limited to palliative, curative, symptom-alleviating, symptom-reducing, disease- or symptom-suppressing, progression-delaying) treatment of cancer.

The term "cancer" means preferably a variety of proliferative diseases, including those mentioned below but are not limited to solid tumors (including benign or especially malign types) such as sarcoma (e.g. Ewing sarcoma, Kaposi's sarcoma or soft part sarcomas such as Dermatofibrosarcoma protuberans), gastrointestinal stromal tumors (GIST), seminoma, carcinoids, mast cell tumors, lung carcinomas, such as small or large cell lung carcinoma, bronchial carcinomas, such as small cell bronchial carcinoma, seminomas, dysgerminomas, testicular intraepithelial neoplasias, melanomas, mammary carcinomas, neuroblastomas, papillary/follicular thyroid carcinoma, malign lymphomas, Non Hodgkin's lymphoma, multiple endocrine neoplasia type 2 (MEN 2), pheochromocytoma, thyroid carcinoma, e.g. medullary thyroid carcinoma, parathyroid hyperplasia/adenoma, mammary carcinoma, colon cancer, colorectal adenoma, ovarian cancer, prostate carcinoma, gliobastoma, brain tumors, prostate carcinoma (also including adenocarcinomas and bone metastasis), malignant gliomas (anaplastic astrocytomas/glioblastomas), pancreatic cancer, malignant pleural mesothelioma, haemangioblastoma, haemangioma, carcinoma of the kidney, liver, adrenal gland, bladder, stomach (especially gastric tumors), rectum, vagina, cervix, endometrium, multiple myeloma, tumors of the head and neck, e.g. squameous carcinoma of the head and neck, including neoplasias, especially of epithelial character, e.g. in the case of mammary carcinoma, malignant nephrosclerosis; or further of other hyperplasias or proliferative diseases.

A "cell which is transformed" as used herein, refers to a prokaryotic or eukaryotic cell that contains heterologous DNA that has been introduced into the cell by any means, e.g. electroporation, calcium phosphate precipitation, microinjection, transformation, viral infection, and the like.

A "vector" is a nucleic acid molecule into which heterologous nucleic acid may be inserted which can then be introduced into an appropriate host cell. Vectors preferably have one or more origin replication and one or more site into which the recombinant DNA can be inserted. Common vectors include plasmids, viral genomes and artificial chromosomes.

A third aspect of the invention encompasses a method for screening an agent for treating cancer which requires bringing a cell which is transformed with an expression vector comprising a polynucleotide encoding a polypeptide consisting of USP26, USP38, USP42 or USP46, a functional fragment or a substantially homologous sequence thereof or a cell membrane thereof into contact with a compound to be tested and analyzing whether or not Mcl-1 is stabilized and selecting an agent destabilizing Mcl-1.

In another aspect, the agent for treating cancer is an inhibitor of the catalytic activity of USPs of the invention. Preferably, in the screening method of the present invention, the agent for treating cancer is an inhibitor of a polypeptide consisting of USP26, USP38, USP42 or USP46, a functional fragment or a substantially homologous sequence thereof.

As used herein, "stabilization" and "destabilization" is related to the half-life assay of Mcl-1.

Another preferred aspect provides a method for screening an agent for treating cancer which requires bringing a cell which is transformed with an expression vector comprising a polynucleotide encoding a polypeptide consisting of USP26, USP38, USP42 or USP46, a functional fragment or a substantially homologous sequence thereof or a cell membrane thereof into contact with a compound to be tested and analyzing the rate of ubiquitination of Mcl-1.

Preferably, the agent for treating cancer is an inhibitor of USPs inducing the increase of ubiquitinated substrate, i.e. ubiquitinated-Mcl-1.

Cell-based systems in drug screening assays can be native or involving recombinant host cells expressing the ubiquitin specific proteases.

The identified agent modulating ubiquitin specific proteases can increase or decrease affinity for ubiquitinated substrate, increase or decrease the rate of binding to a known binding molecule to USP, compete or displace a known binding molecule to the ubiquitin protease.

### FIGURES

Figure 1 (Figures 1.1, 1.2, 1.3) : Schematic representation of Mcl-1 modulation following USP silencing at t=0, 30min or 1h of cycloheximide treatment in HCT116 cells. Mcl-1 increase and decrease are respectively presented in blue and green and siRNA toxicity is presented in grey. Absence of color corresponds to absence of any effect. Selected USP for confirmation experiments are highlighted in green (USP5, USP13, USP19, USP21, USP22, USP26, USP28, USP38, USP42 and USP46). SiRNA designed to target pairs of USP are indicated on the right of the table.
Figure 2 : USP13 screening in HCT116 cells
   Western blot (anti-Mcl-1) revealing expression of endogenous Mcl-1 in the presence of various siRNA targeting either Luc (luciferase, negative control), Mcl-1, (positive control) or USP13 (siRNA13v1, siRNA13v2, siRNA13v3) in HCT116 cells. A weaker exposition is also presented in B. Anti-stat3 antibody was used as loading control. The time course corresponds to the time for which cells were exposed to the protein synthesis inhibitor (cycloheximide) before analysis.

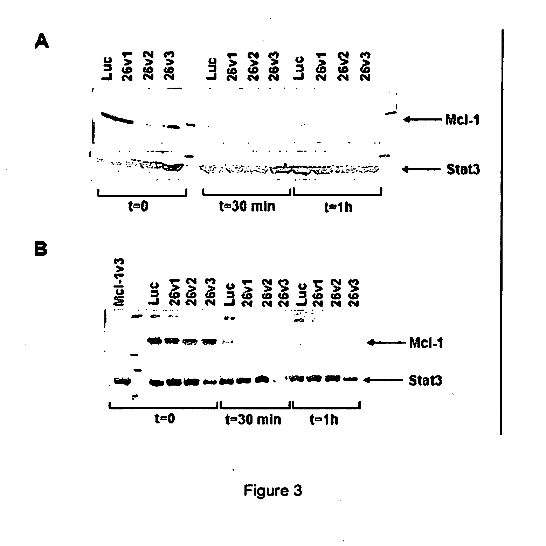

Figure 3 : USP26 screening in HCT116 cells
   Western blot (anti-Mcl-1) revealing expression of endogenous Mcl-1 in the presence of various siRNA targeting either Luc (luciferase, negative control), Mcl-1, (positive control) or USP26 (siRNA26v1, siRNA26v2, siRNA26v3) in HCT116 cells. Anti-stat3 antibody was used as loading control. The time course corresponds to the time for which cells were exposed to the protein synthesis inhibitor (cycloheximide) before analysis.

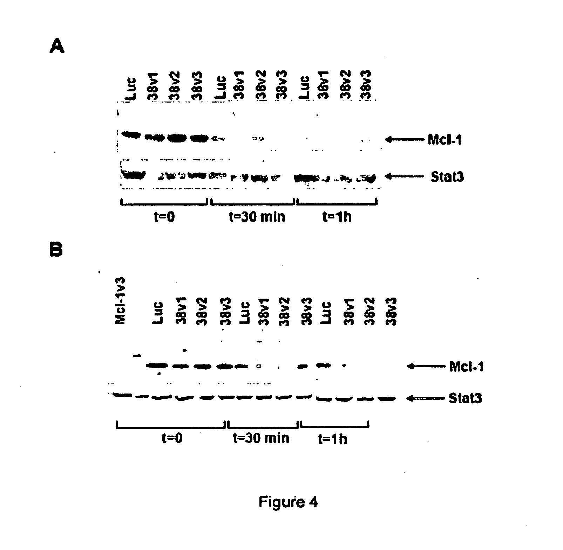

Figure 4 : USP38 screening in HCT116 cells
   Western blot (anti-Mcl-1) revealing expression of endogenous Mcl-1 in the presence of various siRNA targeting either Luc (luciferase, negative control), Mcl-1, (positive control) or USP38 (siRNA38v1, siRNA38v2, siRNA38v3) in HCT116 cells. Anti-stat3 antibody was used as loading control. The time course corresponds to the time for which cells were exposed to the protein synthesis inhibitor (cycloheximide) before analysis.

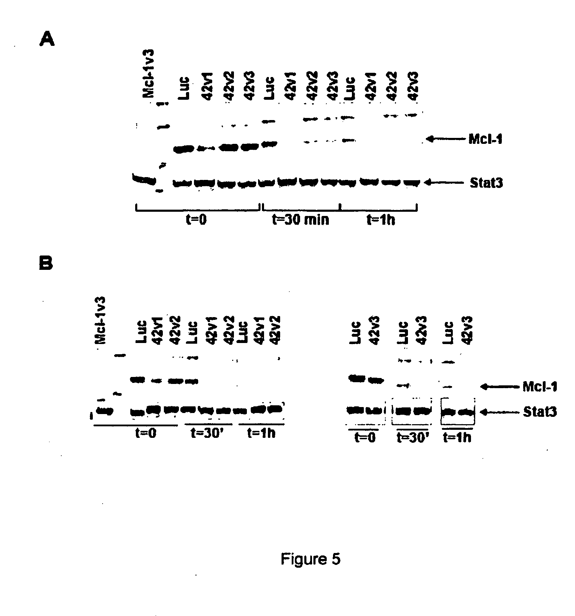

Figure 5 : USP42 screening in HCT116 cells
   Western blot (anti-Mcl-1) revealing expression of endogenous Mcl-1 in the presence of various siRNA targeting either Luc (luciferase, negative control), Mcl-1, (positive control) or USP42 (siRNA42v1, siRNA42v2, siRNA42v3) in HCT116 cells. Anti-stat3 antibody was used as loading control. The time course corresponds to the time for which cells were exposed to the protein synthesis inhibitor (cycloheximide) before analysis.

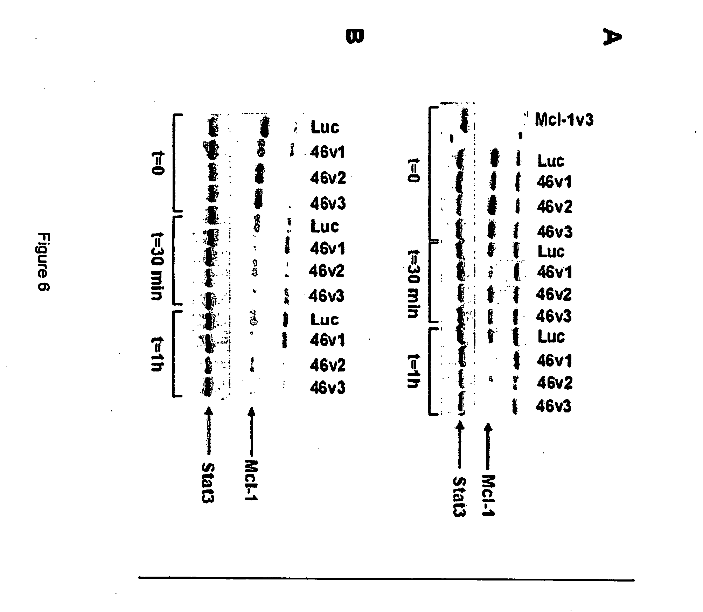

Figure 6 : USP46 screening in HCT116 cells
   Western blot (anti-Mcl-1) revealing expression of endogenous Mcl-1 in the presence of various siRNA targeting either Luc (luciferase, negative control), Mcl-1, (positive control) or USP46 (siRNA46v1, siRNA46v2, siRNA46v3) in HCT116 cells. Anti-stat3 antibody was used as loading control. The time course corresponds to the time for which cells were exposed to the protein synthesis inhibitor (cycloheximide) before analysis.

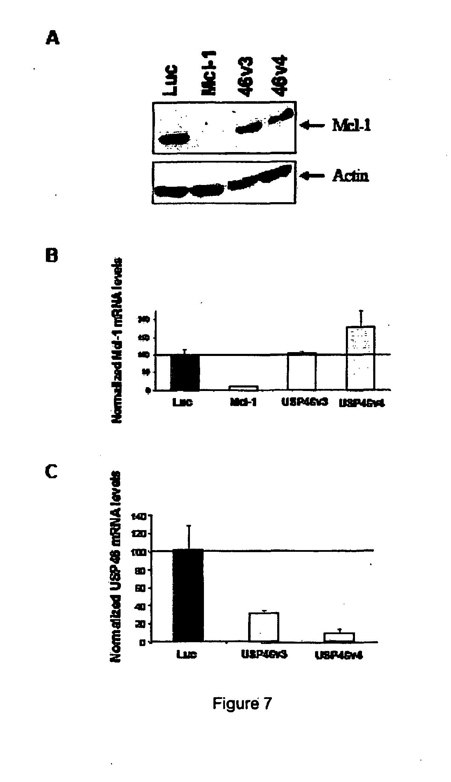

Figure 7 : Phenotype/silencing correlation study in PC3 cells
   Mcl-1 endogenous level was determined in PC3 cells in the presence of several siRNA targeting either luciferase, Mcl-1 or USP46 (A). anti-actin antibody was used as loading control. Mcl-1 (B) and USP46 (C) mRNA levels were determined by quantitative RT-PCR following PC3 transfection with siRNA targeting either luciferase (Luc, negative control), Mcl-1or USP46. The results are shown as means +/- SD of quadruplicate values.

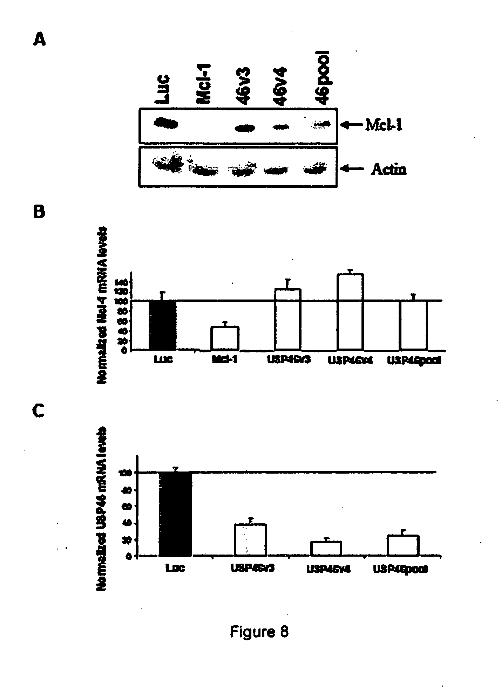

Figure 8 : Phenotype/silencing correlation study in H196 cells
   Mcl-1 endogenous level was determined in H196 cells in the presence of several siRNA targeting either luciferase, Mcl-1 or USP46 (A). anti-actin antibody was used as loading control. Mcl-1 (B) and USP46 (C) mRNA levels were determined by quantitative RT-PCR following H196 transfection with siRNA targeting either luciferase (Luc, negative control), Mcl-1or USP46. The results are shown as means +/- SD of quadruplicate values.

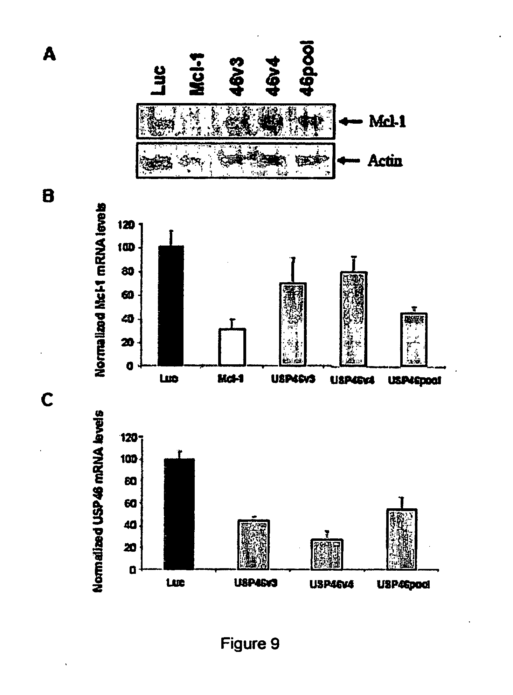

Figure 9 : Phenotype/silencing correlation study in H1703 cells
   Mcl-1 endogenous level was determined in H1703 cells in the presence of several siRNA targeting either luciferase, Mcl-1 or USP46 (A). anti-actin antibody was used as loading control. Mcl-1 (B) and USP46 (C) mRNA levels were determined by quantitative RT-PCR following H1703 transfection with siRNA targeting either luciferase (Luc, negative control), Mcl-1or USP46. The results are shown as means +/- SD of quadruplicate values.

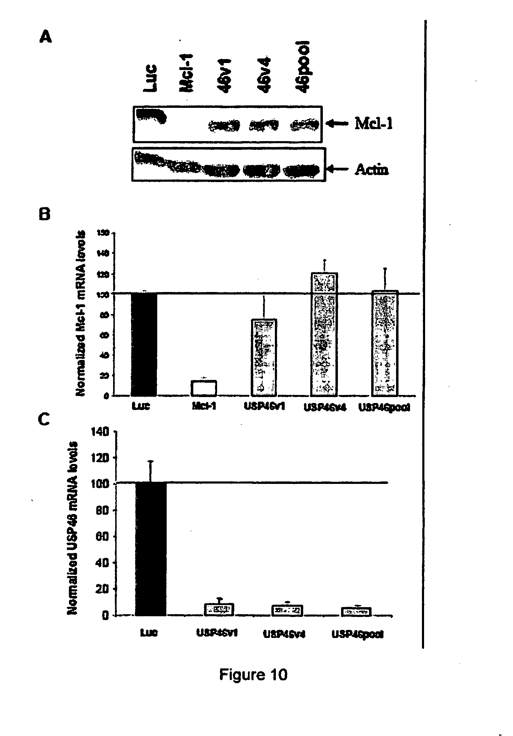

Figure 10 : Phenotype/silencing correlation study in HEK293 cells
   Mcl-1 endogenous level was determined in HEK293 cells in the presence of several siRNA targeting either luciferase, Mcl-1 or USP46 (A). anti-actin antibody was used as loading control. Mcl-1 (B) and USP46 (C) mRNA levels were determined by quantitative RT-PCR following HEK293 transfection with siRNA targeting either luciferase (Luc, negative control), Mcl-1or USP46. The results are shown as means +/- SD of quadruplicate values.

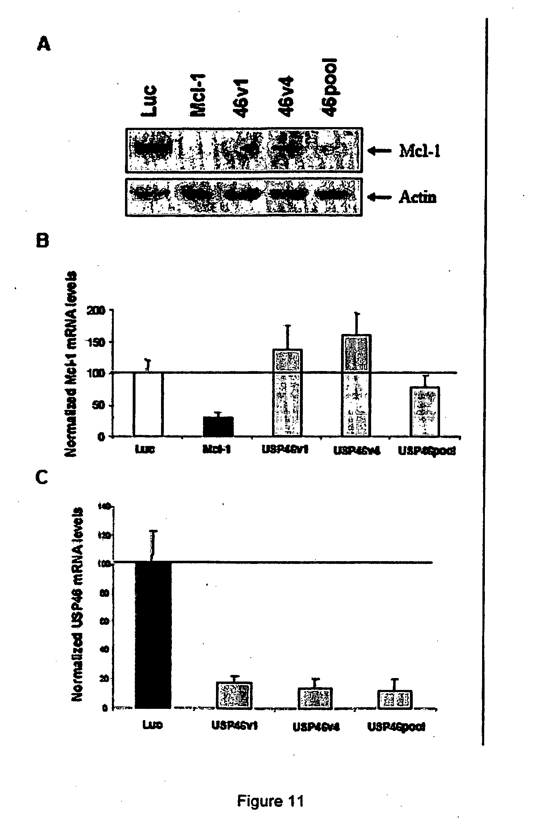

Figure 11 : Phenotype/silencing correlation study in HeLa cells
   Mcl-1 endogenous level was determined in Hela cells in the presence of several siRNA targeting either luciferase, Mcl-1 or USP46 (A). anti-actin antibody was used as loading control. Mcl-1 (B) and USP46 (C) mRNA levels were determined by quantitative RT-PCR following Hela transfection with siRNA targeting either luciferase (Luc, negative control), Mcl-1or USP46. The results are shown as means +/- SD of quadruplicate values.

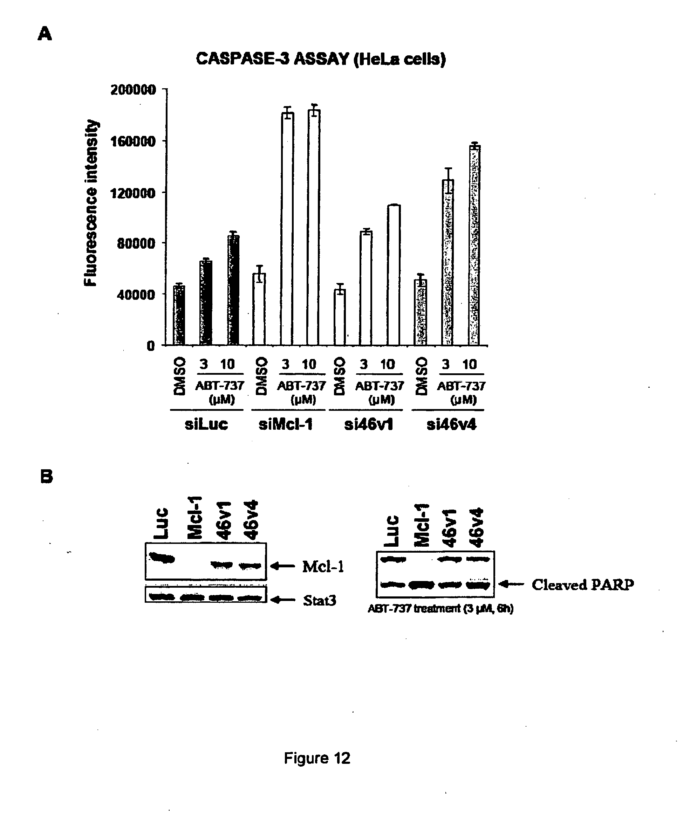

Figure 12 : Effect of USP46 silencing on sensitization of HeLa cells to ABT-737. Caspas 3 assay was used to monitor caspase 3 activity in HeLa cells transfected with siRNA targeting either luciferase (luc, negative control), mcl-1 or USP-46 and treated with DMSO (negative control) or ABT-737 (3-10NM) for 6h. Cell lysates were tested for caspase activity (A). The results are shown as means +/-SD of triplicate values and are representative of two independents experiments. Mcl-1 endogenous level as well as cleaved PARP were also determined in the presence of siRNA targeting either luciferase, Mcl-1 or USP46 (B). Anti-stat3 antibody was used as loading control.

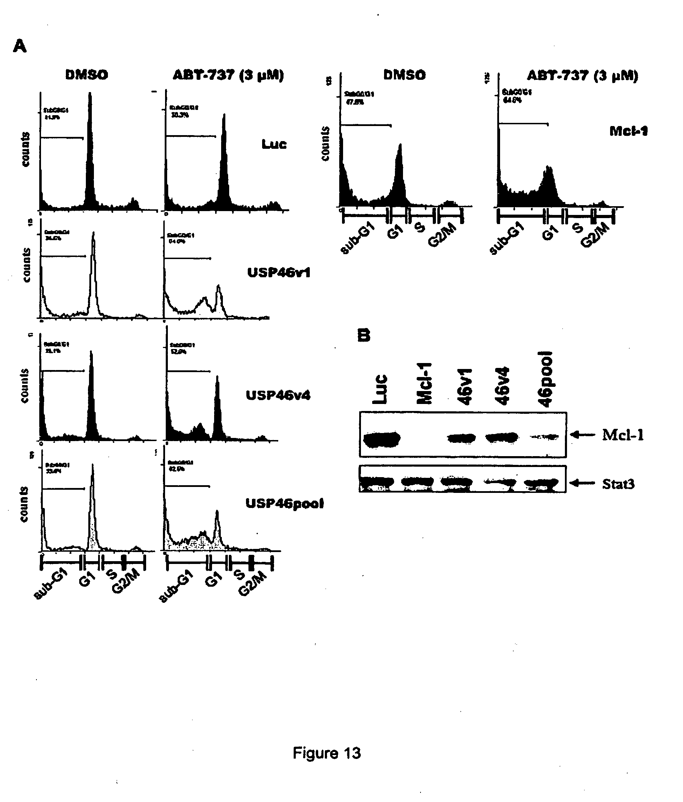

Figure 13 : Flow cytometry of HeLa cancer cell transfected with luc, mcl-1, USP46v1, USP46v4 or USP46 pool siRNA. Cell cycle was determined 72h after siRNA transfection and 18h after ABT-737 treatment (3µM).

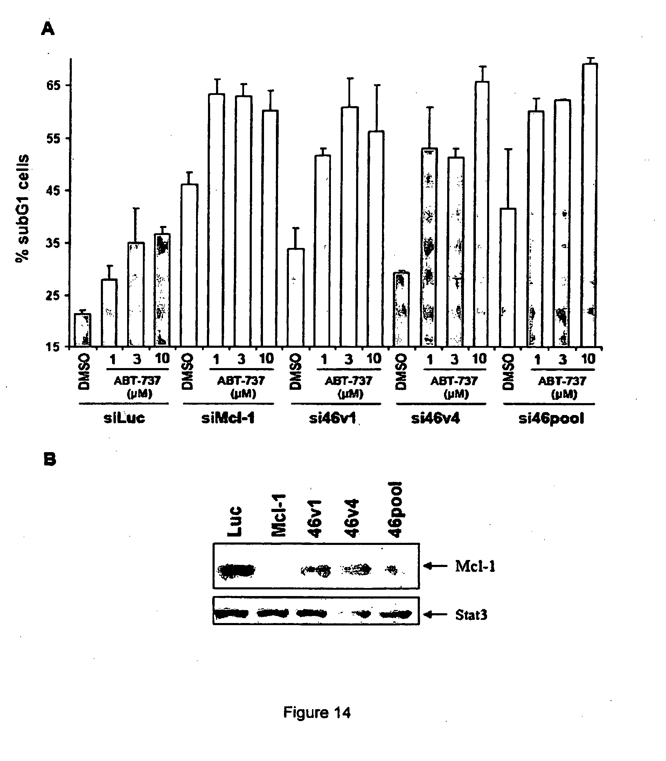

Figure 14 : ABT-737-induced subG1 transition in HeLa cells determined 72h after siRNA transfection and 24h after ABT-737 treatment. The percentage of cells in the subG1 (<2N) are means +/- SD of duplicate values (A). Mcl-1 endogenous level was also determined in the presence of siRNA targeting either luciferase, Mcl-1 or USP46. Anti-Stat3 antibody was used as loading control (B).

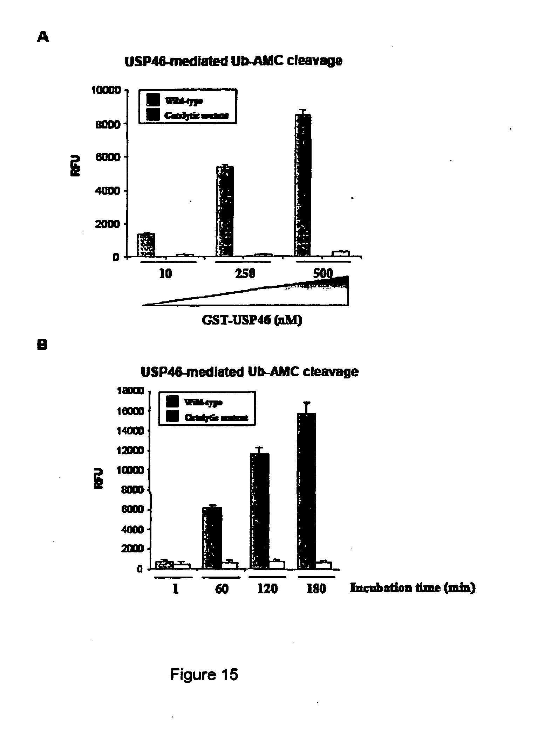

Figure 15 : Deubiquitinating activity of wild-type and catalytic mutant (C44A) GST-USP46 protein purified from E.coli. The enzymatic activity of wild-type and catalytic mutant USP46 protein was evaluated in vitro at various enzyme concentrations and at a fixed incubation time (90min) using UbAMC (400nM) as substrate (A). Kinetics assay of the enzymatic activity of wild-type and catalytic mutant USP46 protein at a fixed concentration of enzyme (400nM) and substrate (800nM) (B). All results are shown as means +/- SD of triplicate values.

### EXAMPLES

### 1. Materials and methods

### 1.1 siRNA design and synthesis

19-mer siRNA synthesized chemically using phosphoramidites were purchased from Proligo. siRNAs were designed as described by Tuschl and colleagues (Elbashir et al. (2001). Duplexes of 21-nucleotide RNAs mediate RNA interference in cultured mammalian cells. Nature 411, 494-8) with particular attention to thermodynamic characteristics (Khvorova et al. (2003). Functional siRNAs and miRNAs exhibit strand bias. Cell 115, 209-16). A tool dedicated to study siRNA specificity was developed in-house. Briefly, NCBI NR and EST databases were requested with each siRNA and target gene by BLAST algorithm. siRNA matching exactly (19/19) or partially (18/19 nucleotides) with an irrelevant NR or EST entry were discarded.

### 1.2 Cell culture and transfection

Human colon carcinoma HCT116 cells were maintained in McCoy's 5A medium containing 10% FBS. The culture media was supplemented with 100 units/ml penicillin and 0.1 mg/ml streptomycin, and cell lines were incubated at 37°C in a humidified atmosphere containing 5% CO₂.
For the screening, the indicated amounts of siRNA (40 pmol, 72 nM final) were complexed with 2µl of Oligofectamine (Invitrogen) in Opti-MEM (Invitrogen) and added to 24-well plates prior to cell seeding. 150 000 HCT116 cells were next plated. Opti-MEM was replaced with appropriate medium 5 hours after transfection. After 72h of transfection and before harvesting, cells were treated or not with cycloheximide (30 µg/ml) for 30 min or 1 hour or treated with MG132 (30µM) for 90 min.

### 1.3 Preparation of Cell Lysates and Western Blot Analysis

Cells were washed once in chilled PBS and harvested by trypsination. Floating and trypsinized cells were pooled and collected by centrifugation. Cell pellets were resuspended in lysis buffer (2% SDS containing 1X protease inhibitor cocktail, Sigma), placed at 100°C for 10 minutes and sonicated for 5 seconds.

Proteins were quantitated using bicinchoninic acid (Sigma-Aldrich) according to manufacturer's instructions. Lysates were resolved by SDS-polyacrylamide (10%) gel electrophoresis (SDS-PAGE), transferred to a nitrocellulose membrane and probed with Mcl-1 rabbit polyclonal antibody (sc-819; SantaCruz), Stat3 rabbit polyclonal antibody (9132; Cell Signaling), Actin mouse monoclonal antibody (A4700; Sigma), or GAPDH mouse monoclonal antibody (Ab8245; AbCam). HRP-conjugated anti-rabbit (7074; Cell Signaling) and HRP conjugated anti-mouse (115-035-003; Jackson ImmunoResearch) antibodies were used as secondary antibodies. Signal was detected using ECL reagent (Amersham) following manufacturer's instructions.

### 1.4 Cell culture, transfection and pharmacological treatment

All cell lines used were maintained in their respective medium. All culture media were supplemented with 100 units/ml penicillin and 0.1 mg/ml streptomycin, and cell lines were incubated at 37°C in a humidified atmosphere containing 5% CO₂. For siRNA transfection, siRNA (40 pmol, 72 nM final) were complexed with 2 µl of Oligofectamine (Invitrogen) in Opti-MEM (Invitrogen) and added to 24-well plates prior to cell seeding. In all conditions, Opti-MEM was replaced with appropriate medium 5 hours after transfection. When Hiperfect was used (Qiagen), siRNA (40 pmol, 72 nM final) were complexed with 3 µl of Hiperfect (Qiagen) in Opti-MEM (Invitrogen) and added to 24-well plates after cell seeding. After 48h or 72h of transfection and before harvesting, cells were treated or not with ABT737 for the indicated time points and at the concentration indicated.
Difficult-to-transfect cells such as H1703 cells were transfected using the AMAXA procedure. Cells are first suspended in special cuvette in the presence of siRNA of interest. An electric pulse, defined according to the cell line used, was next delivered in the Nucleofector device. Nucleofected cell were next plated in 24 or 6-wells plates according to manufacturer's instructions and were treated with ABT737 as described above.

### 1.5 Deubiquitinating assay

Deubiquitinating enzyme activity was monitored in a fluorometric assay, using ubiquitin-AMC or Ub-AMC (ubiquitin-7-amido-4-methylcoumarin, Boston Biochem, U-550) as the substrate, as previously described (Dang et al. (1998) Kinetic and mechanistic studies on the hydrolysis of ubiquitin C-terminal 7-amido-4-methylcoumarin by deubiquitinating enzymes. Biochemistry. 37(7):1868-79). The AMC released from the C-terminus of ubiquitin displays enhanced fluorescence, which can be monitored with a fluorescence reader. Both enzyme and substrate were freshly prepared in USP46 reaction buffer (50 mM Tris-HCl [pH 8.4], 0.5 mM EDTA, 0.05 mg/ml BSA, 5 mM DTT) for each run. The reaction mixture between USP46 and Ub-AMC was incubated at room temperature for 2h and reaction was stopped by adding 10µl of acetic acid (250 mM final concentration). Fluorescence emission intensity was measured on a PHERAstar (BMG Labtech) machine, using a coumarin filter set (λex=360 nm, λem=460 nm).

### 2. Results

### 2.1 USP screening in HTC116 cells

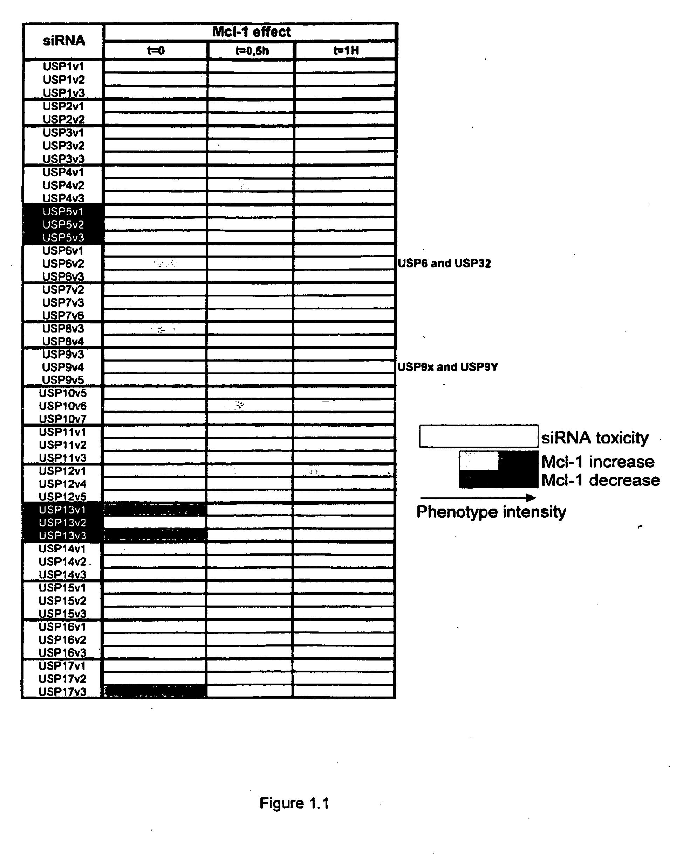

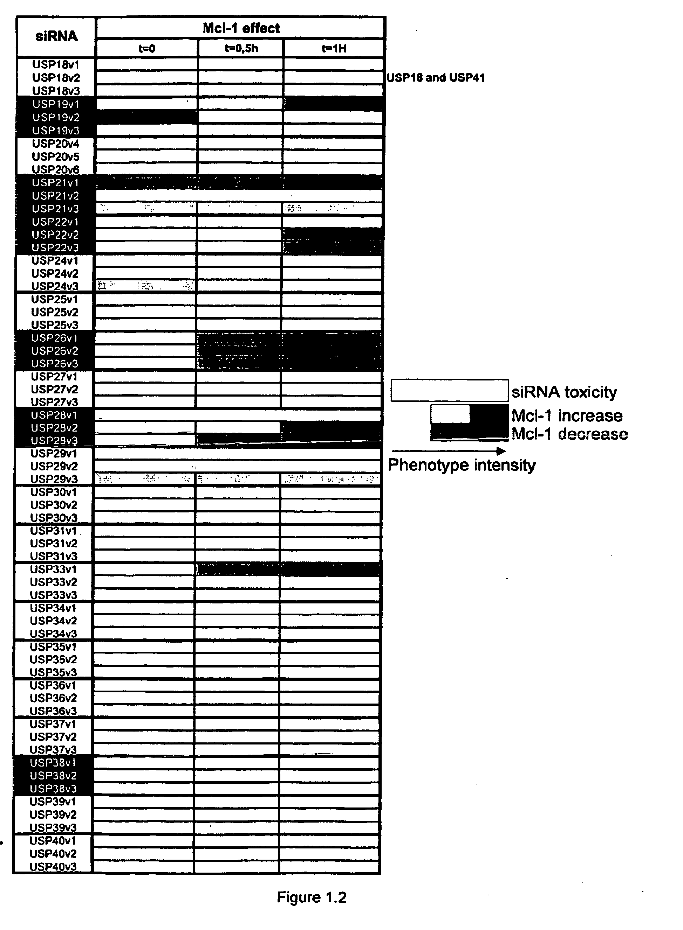

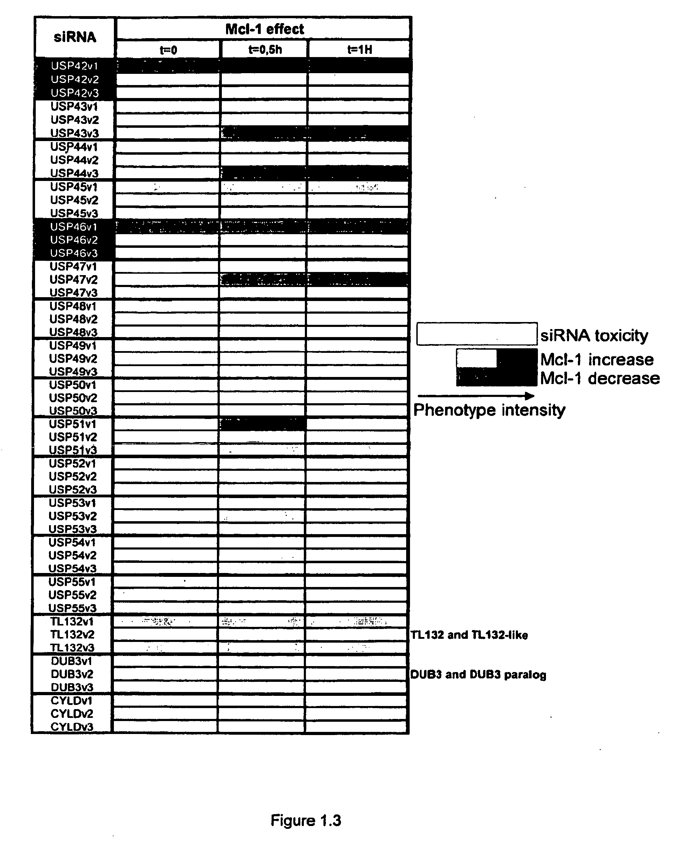

To identify the Ubiquitin Specific Proteases involved in Mcl-1 deubiquitination and thus Mcl-1 stabilization, the entire USP family was screened by RNA interference on Mcl-1 half-life in HCT116 cancer cells. Out of the 60 USP proteins screened, 10 USPs (USP5, USP13, USP19, USP21, USP22, USP26, USP28, USP38, USP42 and USP46) has been identified with the expected phenotype, i.e. a substantial decrease of Mcl-1 (at least 2 out of 3 siRNA exhibiting more than 50% Mcl-1 decrease at one time point). Schematic representation of Mcl-1 modulation following USP silencing and cycloheximide treatment are presented in Figure 1.

### 2.2 Confirmation experiments on selected USP

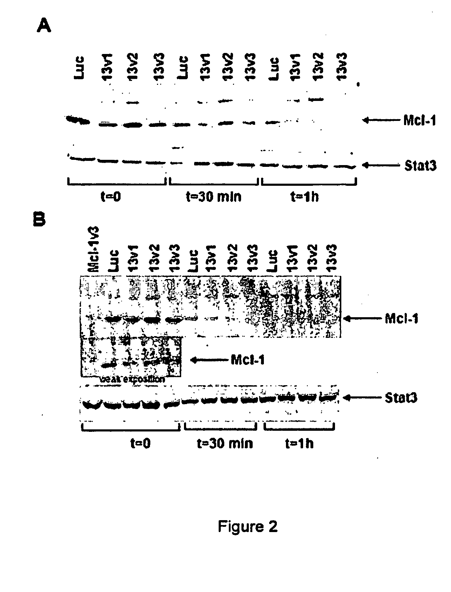

To confirm the results obtained in the primary screen, an independent experiment was done with the 10 selected USPs. The data have been confirmed for 5 USPs (USP13, USP26, USP38, USP42 and USP46) out of 10 USPs of the primary screen results. Western blot results of the confirmation experiments are shown in Figures 2 to 6. These results have disclosed that a significant decrease of endogenous Mcl-1 was observed following cycloheximide treatment (compare Luc siRNA at different timings) and following Mcl-1 silencing. Interestingly, among these proteins, three different USP were shown to have a significant effect on the steady state level of Mcl-1 with 2 siRNA out of 3: USP13, USP42 and USP46.

Therefore, from the screening of the 60 human USPs by RNA interference for Mcl-1 half-life in HCT116 cells, the involvement of 5 USPs (USP13, USP26, USP38, USP42 and USP46) in Mcl-1 stabilization has been confirmed. All these proteins contain amino acids essential for catalytic activity, thus suggesting a functional deubiquitinating activity. Indeed, USP42, USP46 and USP38 have been previously shown to be active in vitro.

### 2.3 Effect of USP46 silencing on Mcl-1 mRNA and protein levels in PC3, H196, H1703, HEK293 and HeLa cells

The effect of Mcl-1 siRNA on endogenous Mcl-1 protein level in PC3, H196, H1703, HEK293 and HeLa cells is monitored and showed a strong decrease of Mcl-1 level, indicating very good transfection efficiency in these cell lines (respectively Figures 7 to 11). In that context, endogenous Mcl-1 protein level was evaluated following USP46 silencing. A significant decrease of Mcl-1 protein level with independent USP46 siRNAs in PC3, H196, H1703, HEK293 and HeLa cells is observed (USP46v3, USP46v4 and pool of active USP46 siRNA) (respectively Figures 7 to 11).
Endogenous Mcl-1 mRNA levels were next determined by quantitative PCR in PC3, H196, H1703, HEK293 and HeLa cells to exclude a transcriptional regulation of Mcl-1 level by USP46. No significant decrease of Mcl-1 mRNA levels following USP46 silencing was observed (respectively Figures 7 to 11) demonstrating that the USP46-mediated Mcl-1 regulation in PC3, H196, H1703, HEK293 and HeLa cells is a post-transcriptional event. Finally, as control, all tested siRNA targeting USP46 affected USP46 mRNA levels :
>68% of decrease in PC3 cells
>62% of decrease in H196 cells
>46% of decrease in H1703 cells
>90% of decrease in HEK293 cells
>80% of decrease in HeLa cells are confirmed (respectively Figures 7 to 11).

These findings show that USP46 is involved in Mcl-1 stabilization in PC3, H196, H1703, HEK293 and HeLa cell lines and thus confirm the previous findings in HCT116 cells.

2.4 Effect of Mcl-1 and USP46 silencing on ABT-737-induced HeLa cell death This assay was used to evaluate the effect of USP46 siRNA on the sensitization of HeLa cells to ABT-737-mediated apoptosis. Our positive control, Mcl-1 siRNA, sensitized cells to ABT-737-mediated apoptosis as shown by a 2.7-fold increase of caspase 3 activity (Figure 12A). Interestingly, USP46v1 and USP46v4 siRNA resulted in significant increase of caspase 3 activity following ABT-737 treatment (v1: 1.5-fold; v4: 2-fold induction at 3 µM ABT-737) (Figure 12A). Importantly, the induction of caspase 3 activity mediated by ABT-737 tightly correlated with the level of Mcl-1 knockdown is observed (Figure 12B). PARP cleavage in the same conditions is also monitored and is observed an increase of cleaved PARP band with Mcl-1 siRNA and an intermediary increase of cleaved PARP band with USP46 siRNA, thus confirming the caspase results (Figure 12B).

To confirm the effect of USP46 siRNA on the sensitization of cells to ABT-737-induced apoptosis in HeLa cells, the number of subG1-phase cells is quantified which reflect mainly apoptosis. Similarly to Mcl-1 siRNA, USP46 siRNAs induced apoptosis in non-treated HeLa cancer cells (Figures 13A and 14A). Indeed, Luc-treated cells presented 21.4 ± 0.6% of subG1 cells whereas Mcl-1-treated cells (46.2 ± 2.2%), USP46v1-treated cells (34 ± 4%), USP46v4-treated cells (29.3 ± 0.3%) and USP46pool-treated cells (40 ± 11%) exhibited higher amount of subG1 cells (Figures 13A and 14A). A slight increase of subG1 cells was observed in the presence of ABT-737 (3 µM) in Luc-treated cells (35% ± 6.6% of subG1 cells) (Figures 13A and 14A). In contrast, a strong increase of subG1 cells was shown when cells were treated with Mcl-1 siRNA in the presence of ABT-737 (63% ± 2% of subG1 cells) (Figures 13A and 14A). Interestingly, USP46v1, USP46v4 as well as a pool of active USP46 siRNA also induced a marked increase of subG1 cells treated with ABT-737 when compared to control siRNA-transfected cells (v1: 60.7% ± 5.4%; v4: 51.3% ± 1.2% pool: 62.4% ± 0.1% of subG1 cells) (Figures 13A and 14A). The degree of subG1 induction in the presence of ABT-737 tightly correlated with the level of Mcl-1 knockdown is observed (Figure 13B).

In conclusion, similarly to Mcl-1 silencing, USP46 silencing decreases Mcl-1 level, induces apoptosis in non-treated HeLa cancer cells and sensitizes HeLa cells to ABT-737-induced cell death. Interestingly, these differences in ABT-737 sensitization always correlated with the silencing efficiency on Mcl-1 level: the more Mcl-1 is decreased following USP46 siRNA transfection, the more USP46-mediated sensitization is observed. USP46 silencing was shown to significantly sensitize cells to ABT-737 at an intermediate level between the negative control (Luc siRNA) and the positive control (Mcl-1 silencing), as evaluated by caspase 3 assay, PARP cleavage and subG1 analysis. Taken together, these results confirm the involvement of USP46 on Mcl-1 regulation in HeLa cervical cancer cells. Similar findings were also observed in HCT116, PC3, H1703 and H196 cells.

### 2.5 In vitro proteolytic activity of USP46

The deubiquitinating activity of both wild-type and mutant purified proteins is compared in vitro. Both wild-type and mutant enzymes were tested in dose-dependent reactions using the Ub-AMC assay (Figure 15). Interestingly, the wild-type USP46 protein hydrolyzed Ub-AMC dose-dependently. In contrast, any dose-dependent effect of catalytically inactive mutant is observed thus indicating the absence of any deubiquitinating activity co-purified with the mutant GST-USP46 (Figure 15A). To confirm these findings, kinetics experiments with GST-USP46 are performed. At a fixed concentration of enzyme and substrate, a time-dependent increase of Ub-AMC hydrolysis with the wild-type USP46 protein but not with the mutant form is observed (Figure 15B). Taken together, these results validate the identification of a specific USP46-mediated deubiquitinating activity with a correct signal/background ratio.

## Claims

1. Use of a USP polypeptide specific to myeloid cell leukemia-1 (Mcl-1) selected from the group consisting of
a) a polypeptide consisting USP26 (ubiquitin specific protease 26), USP38, USP42 or USP46;
b) a functional fragment of a polypeptide USP26, USP38, USP42 or USP46 and exhibiting an activity of deubiquitination;
c) a polypeptide comprising an amino acid sequence having a 84% or more homology with a polypeptide consisting of USP26 (GI: 13994267), USP38 (GI: 31559774), USP42 (GI: 79750943) or USP46 (GI: 31377708) and exhibiting an activity of deubiquitination;
as a screening tool for an agent for treating cancer.

2. Use of a cell which is transformed with an expression vector comprising a polynucleotide encoding a polypeptide as described according to claim 1 and is expressing the polypeptide as a screening tool for an agent for treating cancer.

3. A method for screening an agent for treating cancer comprising the steps of:
a) bringing a cell as described according to claim 2 or a cell membrane thereof into contact with a compound to be tested; and
b) analyzing whether or not Mcl-1 is stabilized;
c) selecting an agent destabilizing Mcl-1.

4. A method for screening an agent for treating cancer comprising the steps of:
a) bringing a cell as described according to claim 2 or a cell membrane thereof into contact with a compound to be tested; and
b) analyzing the rate of ubiquitination of Mcl-1.

## Patentansprüche

1. Verwendung eines USP-Polypeptids, welches für Myeloidzell-Leukämie-1 (Mcl-1) spezifisch ist, gewählt aus der Gruppe bestehend aus:
a) einem Polypeptid bestehend aus USP26 (Ubiquitin-spezifischer Protease 26), USP38, USP42 oder USP46;
b) einem funktionellen Fragment eines Polypeptids bestehend aus USP26, USP38, USP42 oder USP46, wobei das funktionelle Fragment eine Deubiquitinierungs-aktivität aufweist; und
c) einem Polypeptid umfassend eine Aminosäuresequenz mit einer Homologie von 84% oder mehr zu einem Polypeptid bestehend aus USP26 (GI: 13994267), USP38 (GI: 31559774), USP42 (GI: 79750943) oder USP46 (GI: 31377708), wobei das Polypeptid eine Deubiquitinierungsaktivität aufweist,
als ein Screening-Hilfsmittel für ein Mittel zur Behandlung von Krebs.

2. Verwendung einer Zelle, welche mit einem Expressionsvektor transformiert ist, der ein Polynucleotid umfasst, welches ein Polypeptid codiert, wie in Anspruch 1 beschrieben, und welche das Polypeptid als ein Screening-Hilfsmittel für ein Mittel zur Behandlung von Krebs exprimiert.

3. Screening-Verfahren für ein Mittel zur Behandlung von Krebs, umfassend die folgenden Schritte:
a) Inkontaktbringen einer Zelle, wie in Anspruch 2 beschrieben, oder einer Zellmembran davon mit einer zu untersuchenden Verbindung;
b) Untersuchen, ob Mcl-1 stabilisiert wird oder nicht; und
c) Auswählen eines Mittels, welches Mcl-1 destabilisiert.

4. Screening-Verfähren für ein Mittel zur Behandlung von Krebs, umfassend die folgenden Schritte:
a) Inkontaktbringen einer Zelle, wie in Anspruch 2 beschrieben, oder einer Zellmembran davon mit einer zu untersuchenden Verbindung; und
b) Untersuchen der Ubiquitinierungstate von Mcl-1.

## Revendications

1. Utilisation d'un polypeptide USP spécifique de la Mcl-1 (myeloid cell leukemia-1) sélectionné dans le groupe consistant en
a) un polypeptide consistant en USP26 (protéase spécifique de l'ubiquitine 26), USP38, USP42 ou USP46 ;
b) un fragment fonctionnel d'un polypeptide USP26, USP38, USP42 ou USP46 et présentant une activité de déubiquitination ;
c) un polypeptide comprenant une séquence d'acides aminés ayant une homologie d'au moins 84 % avec un polypeptide consistant en USP26 (GI : 13994267), USP38 (GI : 31559774), USP42 (GI : 79750943) ou USP46 (GI : 31377708) et présentant une activité de déubiquitination ;
en tant qu'outil de criblage d'un agent destiné au traitement du cancer.

2. Utilisation d'une cellule qui est transformée avec un vecteur d'expression comprenant un polynucléotide codant pour un polypeptide tel que décrit selon la revendication 1 et qui exprime le polypeptide en tant qu'outil de criblage d'un agent destiné au traitement du cancer.

3. Procédé de criblage d'un agent destiné au traitement du cancer qui comprend les étapes suivantes :
a) mise en contact d'une cellule selon la revendication 2, ou d'une membrane cellulaire de celle-ci, avec un composé à tester ; et
b) analyse du fait que Mcl-1 est ou non stabilisée ;
c) sélection d'un agent déstabilisant Mcl-1.

4. Procédé de criblage d'un agent destiné au traitement du cancer qui comprend les étapes suivantes :
a) mise en contact d'une cellule selon la revendication 2, ou d'une membrane cellulaire de celle-ci, avec un composé à tester ; et
b) analyse du taux d'ubiquitination de Mcl-1.
